# EUROPÄISCHE PATENTSCHRIFT

(11) **EP 0 586 694 B1**
(45) Veröffentlichungstag und Bekanntmachung des Hinweises auf die Patenterteilung: **01.05.1996**
(21) Anmeldenummer: 93917390.2
(22) Anmeldetag: 04.02.1993
(51) Int. Cl.: B05B 15/04, B05B 15/12

(54) **VERFAHREN UND VORRICHTUNG ZUR EMISSIONSARMEN SPRITZAPPLIKATION**
PROCESS AND DEVICE FOR LOW-EMISSION SPRAYING
PROCEDE ET DISPOSITIF DE PEINTURE AU PISTOLET GENERANT PEU D'EMISSIONS

(30) Priorität: 15.02.1992 DE 4204611
(43) Veröffentlichungstag der Anmeldung: 16.03.1994
(73) Patentinhaber: Herberts Gesellschaft mit beschränkter Haftung, D-42285 Wuppertal (DE)
(72) Erfinder: SCHRÖTER, Klaus, D-5600 Wuppertal 2 (DE); TIEGS, Frank-Peter, D-5600 Wuppertal 2 (DE)
(74) Vertreter: Türk, Gille, Hrabal, Leifert
(86) Internationale Anmeldenummer: EP9300255
(87) Internationale Veröffentlichungsnummer: WO9315846

(56) Entgegenhaltungen:
- EP-A- 0 356 733
- CH-A- 473 611
- PATENT ABSTRACTS OF JAPAN vol. 007, no. 056 (C-155)8. März 1983 & JP-A-57 204 264 (KANSAI PAINT KK) 14. Dezember 1982

## Beschreibung

Die Erfindung betrifft ein Verfahren und eine Vorrichtung zur emissionsarmen Spritzapplikation von flüssigen Einkomponentenlacken. Verfahren und Vorrichtung sind besonders geeignet zur Anwendung in der Kraftfahrzeug-Serienlackierung, insbesondere zum Auftrag von Klarlacken.

Im Rahmen steigender Umweltschutzbestrebungen in der Lackiertechnik werden verstärkt Forderungen nach emissionsarmen bzw. emissionsfreien Lacksystemen laut. Gleichzeitig sollen diese Lacke in einem möglichst höhen Maße einem direkten Recycling ohne Aufarbeitung außerhalb der Lackieranlage zu unterziehen sein.

Lacke, die derartige Erfordernisse erfüllen, sind Pulverlacke und flüssige Einkomponentenlacke mit extrem hohem Festkörper von 90 - 100 %. Pulverlacke können in geeigneten geschlossenen Applikationskreisläufen gehandhabt werden, sind jedoch sehr anfällig gegenüber Verunreinigungen. Einfache Methoden zur Korrektur solcher Probleme, wie die im Fall von flüssigen Lacken bekannte Zugabe entsprechender Korrekturadditive oder Filtrationsverfahren, die geeignet sind chemisch inerte Feststoffpartikel mit kleiner Teilchengröße abzutrennen, stehen im Falle der Pulverlacke nicht zur Verfügung. Somit können auch bei der Verwendung von Pulverlacken unerwünschte Lackabfälle entstehen.

Verfahren und Anlagen zur Pulverlackapplikation mit integriertem Direktrecycling sind bekannt. Overspraypulver wird dabei durch Anwendung von Fest/Gasförmig-Trennverfahren aus dem Luftstrom abgetrennt. Beispiele für übliche dazu eingesetzte Aggregate sind Zyklone mit nachgeschaltetem Feinfilter, automatisch im Reverse-Jet-Verfahren reinigungsfähige Kartuschenfilter und in Lackierkabinenwände oder -böden integrierte Filtereinneigen. Diese Filter können durch Absaugen bzw. mechanische Verfahren von anhaftendem Lackpulver befreit werden, welches in der Regel vor dem Wiedereinsatz einer anschließenden Siebung unterworfen werden muß. Dabei muß regelmäßig ein Teil des anwachsenden Feinkornanteils aus cem Kreislauf entfernt werden.

Eine derartige Verfahrensweise und solche Vorrichtungen zur Rückgewinnung sind für den Umgang mit Flüssiglack-Overspray nicht geeignet, da die zur Trennung von Lackaerosol/Luft-Gemisch eingesetzten Filter verkleben würden und nicht durch vorstehend genannte Verfahren von abgetrenntem Lackmaterial befreit werden können.

Im Falle flüssiger Einkomponentenlacke sind Applikationsverfahren mit integriertem Direktrecycling bekannt, z.B. wird in üblichen Lackierkabinen mit Naßauswaschung gearbeitet. Dabei wird die Lackierkabine nicht mit einer üblichen wasserberieselten Innenwand betrieben, sondern anstelle des Wassers wird das Lackmaterial selbst als Auswaschungsmedium umgepumpt. Es ist nur durchführbar für solche hochfestkörperreichen flüssigen Lacksysteme, die eine ausreichend niedrige Viskosität aufweisen. Ein derartiges Verfahren wird in der JP-A-57-204264 beschrieben.

Aufgabe der Erfindung ist die Bereitstellung eines Verfahrens und einer Vorrichtung zur emissionsarmen Spritzapplikation von flüssigen Einkomponentenlacken, die eine Luftverschmutzung weitgehend vermeiden und eine Wiederverwertung des erhaltenen Oversprays ermöglichen. Das Verfahren soll auch für den Einsatz hochviskoser Überzugsmittel geeignet sein. Erfindungsgemäß geschieht dies durch die im kennzeichnenden Teil des Patentanspruchs 1 angegebenen Merkmale.

Es hat sich gezeigt, daß diese Aufgabe gelöst werden kann durch ein Verfahren zur Spritzaoplikation von flüssigen Einkomponentenlacken in einer Lackierkabine, durch die ein Luftstrom geblasen wird, unter Auffangen und Abführen des anfallenden Oversprays, das dadurch gekennzeichnet ist, daß man einen flüssigen Einkomponentenlack mit einem Festkörper über 90 Gew.-% einsetzt, daß man über 80 Gew.-% des Oversprays innerhalb der Lackierkabine an einer oder mehreren Abscheideflächen ohne Naßauswaschung auffängt und von diesen aus der Lackierkabine abführt, daß man die verbleibende Menge des Oversprays aus dem aus der Lackierkabine austretenden Luftstrom mittels eines oder mehrerer Flüssig/Gasförmig-Trennaggregate abtrennt und von diesen abführt, das aus der Lackierkabine und dem Luftstrom abgeführte Overspray ohne weitere Aufbereitung mit frischem Lack zur direkten Wiederverwendung vereint, und daß man den aus der Lackierkabine austretenden Luftstrom im Kreislauf in die Lackierkabine zurückführt.

Durch dieses Verfahren, das einen Gegenstand der Erfindung darstellt, wird es möglich emissionsarme, hochfestkörperreiche Flüssiglacke zu applizieren, wobei ein Direktrecycling des erhaltenen Oversprays stattfindet und eine abluftfreie Verfahrensdurchführung möglich ist. Es ergibt sich der weitere Vorteil, daß ein regelmäßiges Entfernen von Lackmaterial aus dem Recyclingkreislauf vermieden wird. Das aus der Lackierkabine und den Trennaggregaten zurückgewonnene Lackmaterial kann gemeinsam der direkten Wiederverwertung zugeführt werden, ohne daß eine anlagenexterne Aufarbeitung durchgeführt werden muß. Da der Luftstrom in der Lackiervorrichtung im Kreislauf geführt wird, werden Luftverschmutzungen weitgehend vermieden.

Einen weiteren Gegenstand der Erfindung bildet die zur Durchführung des Verfahrens geeignete Vorrichtung, die ein Varratsgefäß für Lack, ein damit verbundenes Sprühorgan, das sich in einer Lackierkabine befindet, die mit Einrichtungen zum Einführen und einer Leitung zum Abführen eines Luftstroms, einer Abscheidezone für Overspray mit Abscheideflächen, ausgerüstet ist, mit außerhalb der Lackierkabine (10) befindlichen, mit der Leitung zum Abführen des Luftstroms verbundenen Flüssig/Gasförmig-Trenneinrichtungen, wobei die Vorrichtung dadurch gekennzeichnet ist, daß die Abscheideflächen der Abscheidezone frei von Einrichtungen zur Naßauswaschung sind, daß die Lackierkabine und die Flüssig/Gasförmig-Trennaggregate jeweils mit Sammelpunkten für Overspray versehen sind, von denen Leitungen zum Vorratsgefäß führen und daß sie eine Leitung zur Rückführung der aus der Lackierkabine abgeführten Luft im Kreislauf zurück in die Lackierkabine aufweist.

Bei dem erfindungsgemäßen Verfahren und bei der erfindungsgemäßen Vorrichtung gelangt der zu applizierende Lack, der bevorzugt ein Klarlack ist, aus einem Vorratstank über eine Versorgungsleitung zum Sprühorgan, das in der Lackierkabine installiert ist und wird dort durch Spritzen, insbesondere Heißspritzen, gegebenenfalls durch elektrostatische Aufladung unterstützt, auf das zu lackierende Objekt aufgebracht. Das Spritzen kann in üblicher Weise, z.B. im Airless-Verfahren, pneumatisch, durch Hochrotationsauftrag oder durch Ultraschallzerstäubung, z.B. Stehwellenzerstäubung erfolgen. Ein Beispiel für eine bevorzugte Heißspritztechnik ist das Hot-Air-Heißspritzen. Im Fall des Heißspritzens wird bei Spritztemperaturen bis zu 90°C, bevorzugt von 35 - 90°C, besonders bevorzugt unter 80°C gearbeitet. Die Temperaturen werden so gewählt, daß ein gutes Verspritzen und ein guter Verlauf sichergestellt wird und bei der kurzzeitig einwirkenden thermischen Belastung keine wesentliche Veränderung des Lackmaterials bzw. des wieder einzusetzenden Oversprays eintritt.

So kann das Heißspritzen beispielsweise so ausgestaltet sein, daß das Lackmaterial nur kurzzeitig in dem oder kurz vor dem Spritzorgan, wie z.B. einer Spritzdüse, erhitzt wird. Es kann z.B. in einem Wärmetauscher geschehen, der in die Systemversorgungsleitung eingebaut ist.

Es ist beispielsweise auch möglich, das Heißspritzen mit superkritischen Lösemitteln zu unterstützen, z.B. gemäß EP-A-0 321 607. Dabei wird in das Überzugsmittel vor der Applikation eine Komponente, die im überkritischen Zustand viskositätserniedrigend wirkt, eingedrückt, wobei die Mischung in den superkritischen Zustand überführt wird, so daß dadurch auf eine geeignete Applikationsviskosität eingestellt werden kann. Ein bevorzugtes Beispiel für eine solche Komponente ist Kohlendioxid. Das Eindrücken und Verspritzen wird gegebenenfalls durch erhöhte Temperaturen unterstützt. Dabei werden die Temperatur oberhalb der kritischen Temperatur und der Druck oberhalb des kritischen Druckes eingestellt. Beim durch superkritische Lösemittel unterstützten Heißspritzverfahren handelt es sich beispielsweise um eine Airless-Spritzapplikation. Im Fall von Kohlendioxid wird beispielsweise bei 35 - 70°C und bevorzugt bei 40 - 60°C gearbeitet. Der Druck liegt bevorzugt bei 90 - 140 bar. Die Obergrenze an Kohlendioxid wird durch die Löslichkeitsgrenze des Lacksystems festgelegt, es darf keine Phasentrennung auftreten. Die Löslichkeitsgrenze ist abhängig von den gewählten Druck- und Temperaturbedingungen. Bevorzugt beträgt die eingesetzte Kohlendioxidmenge, bezogen auf die Gesamtlackmischung, 10 - 50 Gew.-%.

Die superkritische Komponente (z.B. das Kohlendioxid) entweicht bei der Applikation, es ist eine direkte Recyclisierung des Oversprays möglich, da keine Anreicherung der überkritischen Komponente im zurückgeführten Überzugsmittel erfolgt.

Das zu lackierende Objekt befindet sich in der Applikationszone einer Lackierkabine, der eine Abscheidezone nachgeschaltet ist. Diese Abscheidezone kann seitlich oder unterhalb der Applikationszone angeordnet sein, beispielsweise kann oberhalb eines für Overspraynebel durchlässigen Objekttragebodens aus üblichen Gitterrosten lackiert werden.

Das entstandene Overspray schlägt sich zu einem Anteil von über 80 Gew.-% an den Abscheideflächen in der Abscheidezone nieder, von wo nach Aufbau einer von der Viskosität des eingesetzten Lackmaterials und der Temperatur abhängigen Mindestschichtdicke das Lackmaterial nach unten abläuft. Die Abscheideflächen der Abscheidezone können in üblicher Weise, z.B. als deren Wände, Wendungen und/oder Prallbleche, ausgebildet sein. Sie sind beim erfindungsgemäßen Verfahren ohne Naßauswaschung, das heißt es werden keine Auffangflüssigkeiten eingesetzt.

Die Lackierkabine und insbesondere deren Abscheideflächen bestehen bevorzugt aus Edelstahl oder Kunststoff. Sie besitzt Schleusen für den Hinein- und Hinaustransport der zu lackierenden Objekte. Dabei ist es zweckmäßig, Maßnahmen zu treffen, um ein Einschleppen von Verunreinigungen von außerhalb der Lackierkabine zu verhindern, z.B. durch eine gerichtete Luftströmung.

Die Abscheideflächen, z.B. die Wände der Abscheidezone sind bevorzugt aus eine Temperatur beheizbar, die geeignet ist, eine zum Ablaufen von der Innenwand ausreichend niedrige Viskosität des dort niedergeschlagenen Lackmaterials zu gestatten. Je höher die Viskosität des eingesetzten Lackmaterials ist, desto höher wird die Temperatur der Wand gewählt. Die Temperatur wird so niedrig wie möglich gewählt, um das Lackmaterial einer möglichst niedrigen thermischen Belastung auszusetzen. Im allgemeinen werden bevorzugt Wandtemperaturen von 40°C nicht überschritten. Die Beheizung der Abscheideflächen, z.B. der Wände kann kontinuierlich oder diskontinuierlich erfolgen. Die Abscheideflächen können auch mit einem schlecht benetzbaren Material, wie z.B. PVDF beschichtet sein.

In der Abscheidezone der Lackierkabine können sich auch Abscheideflächen in der Form von Prallblechen befinden, die auf bekannte Art und Weise z.B. so angeordnet sein können, daß eine laminare Strömung der Kabinenluft weitgehend vermieden wird. Ein Beispiel für eine Anordnung von Abscheideflächen findet man in der DE-A-40 14 258.

Die im Kreislauf geführte Kabinenluft wird der Lackierkabine im stetigen Strom, bevorzugt von oben her, zugeführt. Die Temperatur der Kabinenluft liegt bevorzugt bei 20 bis 40°C im Fall des Heißspritzens, besonders bevorzugt bei 25 bis 35°C. Es ist zweckmäßig, wenn die Sinkgeschwindigkeit des Luftstromes von der Decke zum Boden der Lackierkabine 0,05 - 0,5 m/Sekunde, bevorzugt 0,3 m/Sekunde nicht übersteigt. Die Vermeidung von Turbulenzen in der Lackierzone kann durch bekannte Maßnahmen, z.B. Verwendung einer üblichen Filterdecke, unterstützt werden.

Der innerhalb der Abscheidezone nicht niedergeschlagene Oversprayanteil von bis zu etwa 20 Gew.-% des gesamten Oversprays wird mit dem Kabinenluftstrom durch eine innerhalb der Abscheidezone angebrachte Rohrverbindung in ein Flüssig/Gasförmig-Trennaggregat befördert und dort aus dem Kabinenluftstrom abgetrennt. Dabei ist es vorteilhaft, wenn die Rohrverbindung mit einem Gefälle versehen ist. Die Flüssig/Gasförmig-Trennaggregate besitzen an der tiefstliegenden Stelle einen Sammelpunkt für abgetrennte Lackoversprayanteile. Beispiele für Flüssig/Gasförmig-Trennaggregate sind nach dem Prinzip der Fliehkraft arbeitende Trennaggregate wie z.B. Zentrifugen und Zyklone oder Elektrofeinfilter. Sie können einzeln oder auch in Kombination eingesetzt werden. Bevorzugt ist die Kombination Zyklon/nachgeschalteter Elektrofeinfilter.

Gegebenenfalls kann noch ein Faserfilter zwecks Entfernung von restlichem Aerosol aus der Kabinenluft nachgeschaltet sein. Die dort gegebenenfalls abgeschiedenen Aerosole machen im allgemeinen nicht mehr als 1 Gew.-% des Gesamtoversprayanfalls aus und können mit den verbrauchten Faserfiltern verworfen werden.

Die von Aerosolen befreite Kabinenluft wird anschließend im Kreislauf zur Kabine zurückgeführt und gegebenenfalls vor Zuführung in die Kabine in einem Wärmetauscher auf die gewünschte Temperatur gebracht. Die Zuführung geschieht nach bekannten Gesichtspunkten zur Erzielung eines gleichmäßigen Luftstroms, bevorzugt über die Kabinendecke.

Das erfindungsgemäße Verfahren arbeitet abluftarm oder bevorzugt abluftfrei. Im Fall des vorstehend erläuterten Einsatzes superkritischer Lösemittel, wie z.B. Kohlendioxid, ist es möglich, einen Teil der Kabinenluft kontinuierlich oder diskontinuierlich auszukreisen, um die gegebenenfalls unerwünschte Anreicherung dieser Gase in der Kabinenluft zu vermeiden. Zweckmäßig geschieht dies nach Passieren des Flüssig/Gasförmig-Trennaggregates, jedoch vor Wiedereinspeisung in die Lackierkabine. In diesem Falle muß das ausgekreiste Teilvolumen der Kabinenluft durch gegebenenfalls konditionierte Frischluft ersetzt werden. Sie kann z.B. zwischen Exhaustor und Lackierkabinendecke eingespeist werden.

Das in der Abscheidezone der Lackierkabine und dem Flüssig/Gasförmig-Trennaggregat sich ansammelnde Lackmaterial kann über eine Sammelleitung in den Lackvorratstank gepumpt werden, wo eine Vermischung mit dem Frischlack gleicher Zusammensetzung erfolgt. Es ist zweckmäßig, wenn das Lackmaterial auf dem Weg zum Vorratstank durch einen Filter geführt wird zur Entfernung von festen Verunreinigungen. Beispiele für geeignete Filter sind übliche Plattenfilter, Kerzenfilter oder Beutelfilter mit Porengrößen von beispielsweise unter 5 µm. Gegebenenfalls kann dem entsprechenden Filterelement ein Wärmeaustauscher vorgeschaltet sein, in dem das Lackmaterial durch Erwärmen auf eine für die Filtration geeignete Viskosität gebracht wird. Die Filtration wird bei Temperaturen von maximal 80°C durchgeführt. Nach Passieren des Filters und vor Eintritt in den Lackvorratstank kann zweckmäßig in einem weiteren Wärmetauscher wieder auf Raumtemperatur abgekühlt werden. Das Filtrationsverfahren kann kontinuierlich durchgeführt werden. Wird es diskontinuierlich durchgeführt, so ist dem Filter ein Behälter zum Sammeln von Lackmaterial vorgeschaltet.

Im Lackvorratstank können gegebenenfalls Korrekturmaßnahmen am Frischlack/Recyclat-Gemisch vorgenommen werden, wie z.B. Zusatz lacküblicher Korrekturadditive oder auch Lösemittel.

Das erfindungsgemäße Verfahren ist geeignet, flüssige Einkomponentenüberzugsmittel mit einem Festkörper von 90 Gew.-% und mehr durch Spritzen, insbesondere Heißspritzen zu applizieren und einem Direktrecycling zu unterwerfen. Die Überzugsmittel besitzen Lösemittelanteile von bis zu 10 %, bevorzugt unter 5 Gew.-%. Besonders bevorzugt sind lösemittelfreie Überzugsmittel. Unter Lösemittel werden in diesem Zusammenhang übliche für Lacke gebräuchliche Lösemittel verstanden, insbesondere solche deren Siedepunkt bzw. Siedepunktbereich 170°C nicht unterschreiten. Beispiele sind Paraffine, z.B. mit C 11 bis C 13; Aromaten, einzeln oder im Gemisch, mit z.B. C 12 bis C 14; oder Ester, z.B. 2,2,4-Trimethyl-1,3-pentandiolmonoisobutyrat.

Die Überzugsmittel können Einbrennsysteme und/oder strahlenhärtende Systeme sein. Im Falle strahlenhärtender Systeme ist es zweckmäßig, das erfindungsgemäße Verfahren unter Lichtausschluß oder so durchzuführen, daß der Zutritt von sichtbarem Licht mit Wellenlängen unter 550 nm wirksam verhindert wird. Die Überzugsmittel müssen ausreichend stabil im Hinblick auf die während des erfindungsgemäßen Verfahrens einwirkenden Temperaturen sein. Sie dürfen sich bei Durchführung des erfindungsgemäßen Verfahrens weder chemisch noch physikalisch wesentlich verändern.

Die erfindungsgemäß applizierten Überzugsmittel können pigmentfrei, aber auch pigmentiert eingesetzt werden. Bevorzugt handelt es sich um Klarlacküberzugsmittel. Beispiele für erfindungsgemäß applizierbare Überzugsmittel sind in der noch nicht veröffentlichten DE-Patentanmeldung DE 42 04 611 beschrieben. Im Folgenden werden die in der genannten DE-Patentanmeldung aufgeführten Überzugsmittel beschrieben. Sie stellen Beispiele und eine bevorzugte Ausführungsform der erfindungsgemäß verwendbaren Überzugsmittel dar. Diese Überzugsmittel enthalten 45 - 85 Gew.-% eines oder mehrerer Polyesterharze (I) sowie 10 - 40 Gew.-% mindestens eines Aminoplastharzes und/oder blockierten Di- und/oder Polyisocyanats als Vernetzer (II), jeweils bezogen auf das gesamte Überzugsmittel, wobei (I) verzweigt aufgebaut und im wesentlichen frei von aromatischen Struktureinheiten ist, eine zahlenmittlere Molmasse (Mₙ) von 350 - 3000 und eine Polydispersität von maximal 3,5 besitzt. Sie weisen eine Viskosität von 100 - 1000 mPa.s, gemessen rotationsviskosimetrisch bei 70°C und einem Schergefälle von 235 s⁻¹ auf. Die Überzugsmittel können gegebenenfalls noch Lösemittel und/oder Reaktivverdünner sowie weitere lacktechnische Additive enthalten.

Die in den bevorzugten Überzugsmitteln eingesetzten Polyesterharze sind verzweigt aufgebaut und enthalten im wesentlichen keine aromatischen Struktureinheiten. Sie besitzen eine Untergrenze der Säurezahl von 12, bevorzugt von 14 mg KOH/g, die Obergrenze der Säurezahl liegt bei 25, bevorzugt bei 20 mg KOH/g, die Untergrenze der Hydroxylzahl beträgt 100, bevorzugt 135, besonders bevorzugt 180 mg KOH/g, die Obergrenze der Hydroxylzahl beträgt 300, bevorzugt 275, besonders bevorzugt 240 mg KOH/g. Bei der Herstellung der Polyesterharze werden die Edukte und die Reaktionsführung so gewählt, daß zahlenmittlere Molekularmassen von 350 bis 3000, bevorzugt von 500 bis 1500 oder von 1000 bis 2000, (Bestimmung durch Gelpermeationschromatographie mit Polystyrolstandard) erhalten werden. Es ist zweckmäßig, wenn die Polydispersität (M_{w}/Mₙ) der Polyesterharze einen Wert von 3,5 nicht überschreitet.

Die Herstellung dieser Polyesterharze erfolgt durch Polykondensation von aliphatischen und/oder cycloaliphatischen, gegebenenfalls ungesättigten, von aromatischen Struktureinheiten freien Mono- und/oder Dicarbonsäuren und/oder deren Anhydriden mit aliphatischen und/oder cycloaliphatischen Mono- und/oder Polyalkoholen. Dabei werden die Mengenanteile der einzelnen Reaktionskomponenten so ausgewählt, daß verzweigte Polyester mit OH-Funktionalitäten von gleich oder größer als 2,3 resultieren. Pro Äquivalent Carboxylgruppe, das sich bis zu 45 % aus Monocarbonsäuren ableiten kann, werden mehr als 0,3 Äquivalente OH-Gruppen, die sich von Polyolen mit mindestens drei OH-Gruppen im Molekül ableiten, eingesetzt. Es wird mit einem OH-Überschuß von mindestens 1,20 Äquivalenten OH pro Äquivalent Carboxylgruppe gearbeitet.

Es können auch zugleich hydroxyl- und carboxylgruppentragende Verbindungen zur Herstellung der Polyesterharze eingesetzt werden, soweit diese Verbindungen frei von aromatischen Struktureinheiten sind. Beispiele dafür sind Dihydroxymonocarbonsäuren wie z.B. Dimethylolpropionsäure.

Bevorzugt werden diese Polyesterharze nach dem dem Fachmann bekannten Schmelzverfahren bei Reaktionstemperaturen von etwa 150 bis etwa 250°C hergestellt.

Die zur Herstellung dieser insbesondere als Klarlacke geeigneten Überzugsmittel verwendeten Polyesterharze werden in Mengenanteilen von 45 - 85 Gew.-%, bevorzugt von 55 - 70 Gew.-%, bezogen auf fertigen Lack, eingesetzt. Sie können auch als Gemisch mehrerer unterschiedlicher Polyesterharze eingesetzt werden, soweit sie nur den vorstehend genannten Definitionen gerecht werden.

Als Vernetzerkomponente (II) werden in den genannten, erfindungsgemäß bevorzugten Überzugsmitteln übliche Aminoplastharze und/oder blockierte Isocyanate eingesetzt. Es handelt sich um teilweise oder vollständig veretherte Amin-Formaldehyd-Kondensationsharze und/oder blockierte Polyisocyanate mit mindestens zwei reaktiven Stellen pro Molekül. Diese Verbindungsklassen sind in der Literatur schon allgemein beschrieben worden.

Diese zur Herstellung der Überzugsmittel verwendeten Vernetzerkomponenten (II) sind vorzugsweise frei von aromatischen Struktureinheiten. Sie werden in Mengenanteilen von 10 bis 40, bevorzugt unter 30 Gew.-% bezogen auf fertiges Überzugsmittel, eingesetzt. Die Verbindungen des Typs (II) können allein oder im Gemisch eingesetzt werden.

Weiterhin können die Überzugsmittel flüssige, niedrigmolekulare, hydroxyfunktionelle Reaktivverdünner enthalten, das heißt Verbindungen, die beim Einbrennvorgang in den Lackfilm chemisch eingebaut werden, mit mindestens zwei Hydroxylgruppen pro Molekül und OH-Zahlen im Bereich von 400 bis 800 mg KOH/g. Geeignet sind Polyole, wie Polyetherpolyole, Polyesterpolyole, Polycarbonatpolyole und Polyurethanpolyole mit Molekularmassen von beispielsweise 150 bis 1000. Geeignet sind beispielsweise Handelsprodukte, wie durch Reaktion von Polyolen mit Caprolacton erhältliche Polycaprolactonpolyole, durch Reaktion von Oxiranverbindungen mit Polyolen und/oder Wasser erhältliche Polyetherpolyole wie z.B. Triethylenglykol, oder durch Reaktion von Polyaminen mit cyclischen Carbonaten erhältliche Polyurethanpolyole.

Die Reaktivverdünner werden in Mengenanteilen von 0 bis 20 Gew.-%, bezogen auf fertiges Überzugsmittel, eingesetzt. Die Obergrenze liegt bevorzugt unter 15 Gew.-%, die Untergrenze beträgt bevorzugt 5, besonders bevorzugt 10 Gew.-%. Die Reaktivverdünner können allein oder im Gemisch eingesetzt werden.

Die erfindungsgemäß eingesetzten Überzugsmittel allgemein und insbesondere die vorstehend beschriebenen bevorzugten Überzugsmittel können Lösemittel in Mengenanteilen bis zu 10, bevorzugt unter 5, besonders bevorzugt unter 3 Gew.-%, bezogen auf fertiges Überzugsmittel, enthalten. Unter Lösemittel werden in diesem Zusammenhang übliche in der Lackindustrie gebräuchliche Lösemittel verstanden,insbesondere solche, deren Siedepunkt bzw. Siedebereich 170°C nicht unterschreitet. Sie sind im Lacksystem inert, das heißt sie werden beim Einbrennvorgang nicht in den Lackfilm chemisch eingebunden. Beispiele sind Paraffine, z.B. mit C₁₁ - C ₁₃; Aromaten, einzeln oder im Gemisch, mit z.B. C ₁₂ - C ₁₄; oder Ester, z.B. 2,2,4-Trimethyl-1,3-pentandiolmonoisobutyrat.

Die genannten, bevorzugt einsetzbaren Überzugsmittel können gegebenenfalls geringe Mengen Vernetzungskatalysator enthalten. Dabei handelt es sich um handelsübliche saure Katalysatoren wie beispielsweise organische Sulfonsäuren, wie p-Toluolsulfonsäure, Dodecylbenzolsulfonsäure, Dinonylnaphthalinsulfonsäure. Die sauren Katalysatoren werden in geblockter Form eingesetzt. Beispiele sind Salze der Sulfonsäuren mit Aminen, wie Diisopropylamin, Morpholin, Pyridin, 2-Amino-2-methyl-1-propanol. Besonders bevorzugt werden die mit Epoxidverbindungen geblockten Sulfonsäuren verwendet, wie z.B. mit Versaticsäureglycidylester geblockte p-Toluolsulfonsäure. Sie werden in üblichen Mengen eingesetzt.

Weiterhin können die Überzugsmittel für Klarlacküberzugsmittel übliche lacktechnische Additive, wie beispielsweise Lichtschutzmittel, Verlaufsmittel, Weichmacher, transparente Pigmente und Füllstoffe, Netzmittel, Antischaummittel, viskositäts- bzw. rheologiebeeinflussende Mittel, enthalten.

Bevorzugt wird bei der Herstellung der vorstehend beschriebenen Überzugsmittel so gearbeitet, daß die Komponente (II) der Komponente (I) bei erhöhter Temperatur zugemischt wird. Die Temperatur wird dabei so gewählt, daß eine Reaktion zwischen den Komponenten (I) und (II) sicher vermieden wird. Die Temperaturen betragen unter 100°C, bevorzugt unter 80°C. Danach können gegebenenfalls anschließend Reaktivverdünner, Lösemittel, Katalysatoren und/oder Additive zugesetzt werden.

Die beschriebenen, erfindungsgemäß einsetzbaren Überzugsmittel sind Einkomponenten-Systeme. Sie zeichnen sich durch eine hohe Lagerstabilität aus und können mehr als sechs Monate ohne wesentliche Viskositätsänderungen gelagert werden.

Als Klarlacküberzugsmittel weisen sie für das Heißspritzverfahren bevorzugt Viskositäten von 100 - 400 mPa.s, besonders bevorzugt von 120 - 200 mPa.s, gemessen rotationsviskosimetrisch bei 70°C und einem Schergefälle von 235 s⁻¹ auf. Das Zahlenmittel des Molekulargewichts (Mₙ) der dafür bevorzugten Bindemittel beträgt 500 bis 1500. Dabei ist die Polydispersität bevorzugt kleiner als 3.0.

Beim Einsatz mit superkritischen Lösemitteln beträgt die Viskosität des unverdünnten, vorstehend beschriebenen bevorzugten Überzugsmittels vorzugsweise 100 bis 1000 mPa.s, rotationsviskosimetrisch gemessen bei 70°C und einem Schergefälle von 235 s⁻¹. Das Zahlenmittel des Molekulargewichts (Mₙ) der Komponente I beträgt für diese Applikationsart bevorzugt 1000 bis 2000.

Die besonders geeigneten Überzugsmittel sind bevorzugt Klarlacküberzugsmittel. Es sind Überzugsmittel, die bei Raumtemperatur eine hohe Viskosität haben können. Sie besitzen einen Lösungsmittelanteil von unter 10 Gew.-%, bevorzugt unter 5 Gew.-%.

Werden pigmentierte, farbige Überzugsmittel eingesetzt, ist es notwendig, um ein direktes Recycling durchführen zu können, daß der Farbton innerhalb der Lackierkabine nicht gewechselt wird. Bei einem Farbtonwechsel sind gründliche Reinigungsarbeiten notwendig, um eine Verunreinigung mit anderen Farbbeimischungen zu verhindern.

Die Überzugsmittel können auf übliche Sustrate, z.B. auf Metall oder Kunststoff, aufgebracht werden. Dabei kann das Substrat direkt beschichtet werden, oder es werden vorher verschiedene Grundierungsschichten aufgetragen. Man erhält auf diese Art und Weise eine Mehrschichtlackierung. Wird ein Klarlacküberzug aufgetragen, so beträgt die Schichtdicke bevorzugt von 20 bis 60 µm, besonders bevorzugt von 35 bis 50 µm. Als Basisschicht wird bevorzugt ein farbiger Effektbasislack, bevorzugt ein Wasserbasislack, aufgetragen. Dabei kann gegebenenfalls naß-in-naß gearbeitet werden oder der Basislack wird vorher durch Erwärmen getrocknet. Es kann vorteilhaft sein, wenn das zu lackierende Substrat beim erfindungsgemäßen Heißspritzauftrag eine Temperatur von bis zu 50°C besitzt. Die Temperatur kann beispielsweise von vorherigen Arbeitsschritten, z.B. Trocknungs- und Einbrennschritten, herrühren. Es ist jedoch auch möglich, daß das Substrat an einer Wärmequelle vorbei, z.B. einer Heißluftblaszone oder einem Infrarotstrahler, geführt wird und dabei auf eine höhere Temperatur erwärmt wird.

Nach dem Beschichten wird der Lackfilm durch erhöhte Temperatur und/oder Bestrahlen vernetzt. Die Einbrenntemperaturen der Überzugsmittel, insbesondere der vorstehend als bevorzugt beschriebenen, liegen beispielsweise bei 130 - 170°C, bevorzugt bei 140 bis 160°C.

Das erfindungsgemäße Verfahren und die erfindungsgemäße Vorrichtung eignen sich besonders zur Anwendung in der Kraftfahrzeug-Serienlackierung, beispielsweise zur Lackierung von Automobilkarossen und deren Teilen; sie können jedoch auch für andere Zwecke verwendet werden, wie z.B. bei der Lackierung von Haushaltsgeräten oder Möbeln.

Das erfindungsgemäße Verfahren und die erfindungsgemäße Vorrichtung zeichnen sich durch hohe Wirtschaftlichkeit aus und sind von hohem ökologischen Wert. Sie erlauben emissionsarmes Arbeiten, insbesondere werden nur geringe Lösemittelemissionen oder geringe Mengen an Prozeßabluft abgegeben. Nur geringe Frischluftmengen müssen konditioniert werden. Es werden dabei Lackabfälle weitgehend vermieden.

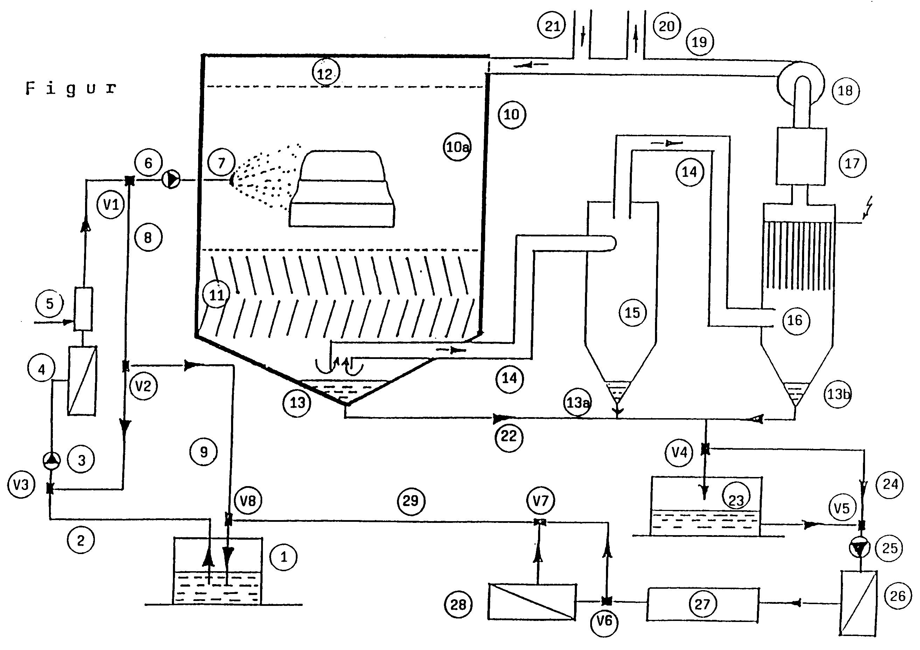

Die beigefügte Figur stellt ein Beispiel der vorliegenden Erfindung, das das schematische Verfahren und die Vorrichtung erläutert. Das Überzugsmittel befindet sich in einem Vorratstank (1), über den es durch die Systemversorgungsleitung (2), über die Pumpe (3) und das Ventil (V1) zur Dosierpumpe (6) und von dort zum Sprühorgan (7) geführt wird. Um das Überzugsmittel auf Prozeßtemperatur zu bringen, kann ein Wärmetauscher (4) vorgesehen sein, hinter den eine für die gegebenenfalls gewünschte Zumischung eines superkritischen Lösemittels geeignete Mischstation (5) geschaltet sein kann. Ist die Dosierpumpe (6) nicht aktiv, so leitet das Ventil (V1) das Überzugsmittel über die Rückführungsleitung (8) und über die Ventile (V2) und (V3) in die Systemversorgungsleitung (2) zurück. Das Ventil (V2) kann auch so geschaltet sein, daß das Überzugsmittel über die Leitung (9) und das Ventil (V8) in den Vorratstank (1) zurückgeführt wird.

In der Applikationszone (10a) der Lackierkabine (10) wird das Überzugsmittel nach bekannten Verfahren mittels Sprühorgan (7) auf das Substrat aufgetragen.

Die Lackierkabine (10) enthält das Sprühorgan (7) innerhalb der Applikationszone (10a), die der Luftzuführung dienende Kabinendecke (12) sowie als tiefstliegende Stelle der Prallflächen enthaltenden Abscheidezone (11) den Sammelpunkt (13), in dem das sich innerhalb der Abscheidezone niederschlagende Lackmaterial zusammenfließt. Die Wände der Abscheidezone (11) sind bevorzugt beheizbar ausgeführt. Aus der Abscheidezone (11) führt eine gegebenenfalls mit Gefälle versehene Rohrleitung (14) in die Flüssig-Gasförmig-Trennvorrichtung (15). Diese kann beispielsweise ein Zyklon sein, welcher an der tiefstliegenden Stelle ebenfalls einen Sammelpunkt (13a) für das Lackmaterial besitzt. Vom Zyklon (15) führt eine weitere gegebenenfalls mit Gefälle versehene Rohrleitung (14) zu einem Elektrofilter (16), der an der tiefstliegenden Stelle ebenfalls einen Sammelpunkt (13b) für abgeschiedenes Lackmaterial besitzt. Nach Passieren des Elektrofilters (16) und eines gegebenenfalls nachgeschalteten Feinstfilters (Faserfilter, 17), wird die gereinigte, filtrierte Luft über einen Exhaustor (18) und die Luftleitung (19) zur Lackierkabinendecke (12) zurückgeführt. Dabei kann gegebenenfalls eine Abzweigung (20) zum Auskreisen überschüssiger Abluftanteile sowie gegebenenfalls eine zur Einsteuerung von konditionierter Frischluft geeignete Zuführung (21) vorgesehen sein.

Das an den Sammelpunkten (13), (13a) und (13b) zurückgewonnene Lackmaterial gelangt über das Leitungssystem (22) bei diskontinuierlicher Fahrweise über das Ventil (V4) in den Sammeltank (23) und von dort über das Ventil (V5) und die Pumpe (25), den gegebenenfalls vorhandenen Wärmetauscher (26) und den zur Entfernung fester Verunreinigungen geeigneten Lackfilter (27) zu den Ventilen (V6 und V7) und gelangt von dort direkt oder nach Passieren des gegebenenfalls vorhandenen Wärmetauschers (28) über die Leitung (29) und das Ventil (V8) zurück in den Vorratstank (1). Bei kontinuierlicher Fahrweise umgeht der Lackstrom über die Leitung (24) den Sammeltank (23) und wird über das Ventil (V5) zur Pumpe (25) geführt.

Die in der schematischen Zeichnung dargestellte Lackieranlage bildet nur ein Beispiel. Die verschiedenen Aggregate und ihre Anordnung können durch äquivalente Maßnahmen vom Fachmann leicht ersetzt bzw. verändert werden.

Im Folgenden sind einige Beispiele für Überzugsmittel, insbesondere Klarlacküberzugsmittel angegeben, die bevorzugt für das erfindungsgemäße Verfahren eingesetzt werden können.

### Beispiel 1:

In einer für die Polyestersynthese geeigneten Reaktionsapparatur wurden 287 g Neopentylglykol, 57 g Ethylenglykol, 134 g Trimethylolpropan, 197 g Adipinsäure, 324 g Hexahydrophthalsäureanhydrid und 1 g hypophosphorige Säure langsam aufgeschmolzen und innig vermischt. Nach Erreichen von 170°C setzte Polykondensation unter Wasserabspaltung ein, wobei die Temperatur langsam auf maximal 250°C gesteigert wurde, so daß Wasser kontinuierlich abdestillierte, ohne daß die Kolonnenkopftemperatur 100°C überschritt. Nach Erreichen einer Säurezahl von 16 mg KOH/g wurde rasch abgekühlt.

Man erhielt einen Polyester mit einer OH-Zahl von 229 mg KOH/g und einer zahlenmittleren Molekularmasse von 800 (gelpermeationschromatographisch, geeicht mit Polystyrolstandard). Die Polydispersität betrug 2,5.

Zu 751,5 g des erhaltenen Polyesters wurden nach Abkühlen auf 70°C 225,5 g Hexamethoxymethylmelamin (Polymerisationsgrad: 1,5) gegeben, gut vermischt und nach Abkühlen auf 60°C mit 23 g Texanol vermischt.

79,42 Teile der vorstehend genannten Harzmischung wurden unter Rühren nacheinander mit 5,83 Teilen Triethylenglykol, 5,83 Teilen eines handelsüblichen Polycaprolactontriols mit einer zahlenmittleren Molmasse von 300, 2,91 Teilen des Diesters aus Phthalsäure und 2-Ethylhexanol, 1,75 Teilen Versaticsäureglycidylester-geblockter p-Toluolsulfonsäure, 1,46 Teilen eines Gemischs handelsüblicher Verlaufsmittel auf Polysiloxanbasis, 0,78 Teilen eines handelsüblichen nichtsäurebindenden Lichtschutzmittels auf HALS-Basis, 1,55 Teilen eines handelsüblichen Lichtschutzmittels auf Benzotriazol-Basis und 0,47 Teilen eines handelsüblichen Netzmittels auf Basis eines niedermolekularen Acrylatharzes vermischt.

Man erhielt ein Klarlacküberzugsmittel mit einer Viskosität von 160 mPa.s, gemessen im Rotationsviskosimeter bei 70°C und einem Schergefälle von 235 s⁻¹.

### Beispiel 2:

Analog zu Beispiel 1 wurden 368 g Neopentylglykol, 129 g Trimethylolpropan, 189 g Adipinsäure, 312 g Hexahydrophthalsäureanhydrid und 1 g hypophosphorige Säure polykondensiert, bis eine Säurezahl von 13,4 mg KOH/g erreicht war.

Der erhaltene Polyester hatte eine OH-Zahl von 200 mg KOH/g und eine zahlenmittlere Molekularmasse von 800 (gelpermeationschromatographisch, geeicht mit Polystyrolstandard) bei einer Polydispersität von 2,4.

Bei 70°C wurden 752,1 g des Polyesters mit 225 g Hexamethoxymethylmelamin (Polymerisationsgrad: 1,5) und 22,9 g Texanol vermischt.

82,61 Teile dieser Harzmischung wurden mit 5,65 Teilen Triethylenglykol, 6 Teilen des in Beispiel 1 genannten Polycaprolactontriols, 1,76 Teilen der geblockten Sulfonsäure gemäß Beispiel 1, 2,42 Teilen einer Mischung handelsüblicher Verlaufsmittel auf Polysiloxanbasis und jeweils 0,78 Teilen der in Beispiel 1 genannten Lichtschutzmittel innig vermischt. Man erhielt ein Klarlacküberzugsmittel mit einer Viskosität von 175 mPa.s, gemessen im Rotationsviskosimeter bei 70°C und einem Schergefälle von 235 s⁻¹.

### Beispiel 3:

Analog Beispiel 1 wurden 199 g Neopentylglykol, 119 g Ethylenglykol, 139 g Trimethylolpropan, 205 g Adipinsäure, 337 g Hexahydrophthalsäureanhydrid und 1 g hypophosphorige Säure polykondensiert, bis eine Säurezahl von 16 mg KOH/g erreicht war. Der erhaltene Polyester hatte eine OH-Zahl von 206 mg KOH/g und eine zahlenmittlere Molekularmasse von 800 (gelpermeationschromatographisch, Polystyrolstandard) bei einer Polydispersität von 2,4.

Bei 70°C wurden 752,1 g des Polyesters mit 225 g Hexamethoxymethylmelamin (Polymerisationsgrad: 1,5) und 22,9 g Texanol vermischt.

81 Teile dieser Mischung wurden mit 6 Teilen Triethylenglykol, 6 Teilen des in Beispiel 1 genannten Polycaprolactontriols, 1,7 Teilen der in Beispiel 1 aufgeführten blockierten Sulfonsäure, 3 Teilen einer Mischung handelsüblicher Verlaufsmittel auf Polysiloxanbasis, jeweils 1 Teil der in Beispiel 1 genannten Lichtschutzmittel und 0,3 Teilen des in Beispiel 1 genannten Netzmittels unter Rühren innig vermischt.

Man erhielt ein Klarlacküberzugsmittel mit einer Viskosität von 146 mPa.s, gemassen im Rotationsviskosimeter bei 70°C und einem Schergefälle von 235 s⁻¹.

### Beispiel 4:

Analog zu Beispiel 1 wurden 431,1 g Pentaerythrit, 450,8 g Hexahydrophthalsäureanhydrid, 616,6 g Isononansäure und 1,5 g hypophosphorige Säure polykondensiert, bis eine Säurezahl von 18 mg KOH/g erreicht war.

Der erhaltene Polyester hatte eine OH-Zahl von 137 mg KOH/g und ein zahlenmittleres Molekulargewicht von 1900 (gelpermeationschromatographisch, Polystyrolstandard) bei einer Polydispersität von 3,2.

Bei 70°C wurden 750 g des Polyesters mit 227 g Hexamethoxymethylmelamin (Polymerisationsgrad 1,5) und 23 g Shellsol R (aromatischer Kohlenwasserstoff) vermischt. 77,6 Teile dieser Harzmischung wurden mit 7 Teilen Triethylenglykol, 6,5 Teilen des in Beispiel 1 beschriebenen Polycaprolactontriols, 1,5 Teilen des in Beispiel 1 genannten Phthalsäurediesters, 1,8 Teilen der geblockten Sulfonsäure gemäß Beispiel 1, 3 Teilen des Gemisches handelsüblicher Verlaufsmittel gemäß Beispiel 1, 0,5 Teilen des HALS-Lichtschutzmittels gemäß Beispiel 1, 1,6 Teilen des Benzotriazol-Lichtschutzmittels gemäß Beispiel 1 und 0,5 Teilen des Netzmittels gemäß Beispiel 1 vermischt.

Man erhielt ein Klarlacküberzugsmittel mit einer Viskosität von 490 mPa.s, gemessen im Rotationsviskosimeter bei 70°C und einem Schergefälle von 235 s⁻¹.

## Patentansprüche

1. Verfahren zur Spritzapplikation von flüssigen Einkomponentenlacken, in einer Lackierkabine, durch die ein Luftstrom geblasen wird, unter Auffangen und Abführen des anfallenden Oversprays, wobei man einen Teil des Oversprays innerhalb der Lackierkabine an einer oder mehreren Abscheideflächen ohne Naßauswaschung auffängt und von diesen aus der Lackierkabine abführt, die verbleibende Menge des Oversprays aus dem aus der Lackierkabine austretenden Luftstrom mittels eines oder mehrerer Flüssig/Gasförmig-Trennaggregate abtrennt und von diesen abführt, das aus der Lackierkabine und dem Luftstrom abgeführte Overspray ohne weitere Aufbereitung mit frischem Lack zur direkten Wiederverwendung vereint und den aus der Lackierkabine austretenden Luftstrom im Kreislauf in die Lackierkabine zurückführt, dadurch gekennzeichnet, daß man mit einem heißspritzbaren Einkomponentenlack bei Temperaturen bis zu 90°C mit einem Festkörper über 90 Gew.-% spritzt, über 80 Gew.-% des Oversprays abführt und in einer Lackierkabine mit beheizten Abscheideflächen arbeitet.

2. Verfahren nach Anspruch 1, dadurch gekennzeichnet, daß man das aus der Lackierkabine abgeführte Overspray vor dem Vermischen mit frischem Lack filtriert.

3. Verfahren nach einem der Ansprüche 1 oder 2, dadurch gekennzeichnet, daß man den Lack mit Hilfe eines superkritischen Lösemittels, insbesondere Kohlendioxid, spritzt.

4. Verfahren nach einem der vorhergehenden Ansprüche, dadurch gekennzeichnet, daß man mit einem Lack mit einem Anteil oder mehreren flüssigen organischen Lösemitteln unter 5 Gew.-% arbeitet.

5. Verfahren nach einem der vorhergehenden Ansprüche, dadurch gekennzeichnet, daß man mit einem Lack mit einem Gehalt eines oder mehrerer flüssiger organischer Lösemittel mit einem Siedepunkt bzw. Siedepunktbereich von 170°C und darüber, arbeitet.

6. Verfahren nach einem der Ansprüche 1 bis 3, dadurch gekennzeichnet, daß man mit einem Lack arbeitet, der frei von flüssigen organische Lösemitteln ist.

7. Verfahren nach einem der vorhergehenden Ansprüche, dadurch gekennzeichnet, daß man mit einem Einkomponenten-Klarlack arbeitet.

8. Verfahren nach einem der vorhergehenden Ansprüche, dadurch gekennzeichnet, daß als Lack ein strahlenhärtbares Überzugsmittel verwendet wird.

9. Verfahren nach einem der Ansprüche 1 bis 7, dadurch gekennzeichnet, daß mit einem durch Einbrennen härtbaren Lack gearbeitet wird.

10. Verfahren nach Anspruch 9, dadurch gekennzeichnet, daß man mit einem heißspritzbaren Lack, insbesondere einem Klarlack, arbeitet, auf der Basis von
I. 45 bis 85 Gew.-% eines oder mehrerer Polyester, die verzweigt aufgebaut und im wesentlichen frei von aromatischen Struktureinheiten sind, mit einem zahlenmittleren Molekulargewicht von 350 bis 3000 und einer Polydispersität von kleiner als 3,5, und
II. 10 bis 40 Gew.-% eines oder mehrerer Aminoplastharze und/oder blockierter Di- und/oder Polyisocyanate als Vernetzer, und
0 bis 20 Gew.-% eines oder mehrerer Reaktivverdünner, und
0 bis 10 Gew.-% eines oder mehrerer organischer Lösemittel, wobei der Lack eine Viskosität von 100 bis 1000 mPa.s, rotationsviskosimetrisch gemessen bei 70°C und einem Schergefälle von 235 s⁻¹, aufweist.

11. Vorrichtung, geeignet zur Durchführung des Verfahrens nach einem der Ansprüche 1 bis 10, mit einem Vorratsgefäß (1) für Lack, einem damit verbundenen Sprühorgan (7), das sich in einer Lackierkabine (10) befindet, die mit Einrichtungen zum Einführen (12) und einer Leitung (14) zum Abführen eines Luftstromes, einer Abscheidezone (11) für Overspray mit Abscheideflächen, ausgerüstet ist, mit außerhalb der Lackierkabine (10) befindlichen, mit der Leitung (14) verbundenen Flüssig/Gasförmig-Trenneinrichtungen (15,16), wobei die Abscheideflächen der Abscheidezone (11) frei von Einrichtungen zur Naßauswaschung sind, die Lackierkabine (10) und die Flüssig/Gasförmig-Trenneinrichtungen (15,16) mit Sammelpunkten (13,13a,13b) für Overspray versehen sind, von denen Leitungen (22,24,29) zum Vorratsgefäß (1) führen, und daß eine Leitung (19) zur Rückführung der durch die Leitung (14) aus der Lackierkabine (10) abgeführten Luft im Kreislauf in die Lackierkabine (10) vorgesehen ist, dadurch gekennzeichnet, daß die Abscheideflächen der Abscheidezone (11) beheizbar sind.

12. Vorrichtung nach Anspruch 11, dadurch gekennzeichnet, daß die aus den Sammelpunkten (13,13a13b) abführende Leitung (22) zu einem Sammeltank (23) führt, der mit dem Vorratstank (1) verbunden ist.

13. Vorrichtung nach einem der Ansprüche 11 und 12, dadurch gekennzeichnet, daß in die Sammelpunkte (13,13a13b) und den Vorratstank (1) verbindende Leitung (22,24,29) ein oder mehrere Wärmeaustauscher (26,28) und/oder Lackfilter (27) einbezogen sind.

## Claims

1. A process for the spray application of liquid single-component lacquers in a spray booth through which an air current is blown, with the capture and discharge of the overspray arising, wherein part of the overspray is captured inside the spray booth on one or more deposition surfaces without wet scrubbing and is discharged from the latter out of the spray booth, the remaining amount of overspray is separated from the air current emerging from the spray booth by means of one or more liquid/gas separator units and is discharged from the latter, the overspray discharged from the spray booth and the air current is combined with fresh lacquer for direct reuse without further processing and the air current emerging from the spray booth is fed back in circulation to the spray booth, characterised in that spraying is effected with a hot-sprayable single-component lacquer at temperatures up to 90°C with a solids content greater than 90 % by weight, more than 80 % by weight of the overspray is discharged, and a spray booth with heated deposition surfaces is employed.

2. A process according to claim 1, characterised in that overspray discharged from the spray booth is filtered before mixing with fresh lacquer.

3. A process according to either one of claims 1 or 2, characterised in that the lacquer is sprayed with the aid of a supercritical solvent, particularly carbon dioxide.

4. A process according to any one of the preceding claims, characterised in that lacquer is employed which has a content of one or more liquid organic solvents less than 5 % by weight.

5. A process according to one of the preceding claims, characterised in that a lacquer is employed which has a content of one or more liquid organic solvents with a boiling point or boiling point range of 170°C and above.

6. A process according to any one of claims 1 to 3, characterised in that a lacquer is employed which is free from liquid organic solvents.

7. A process according to any one of the preceding claims, characterised in that a single-component clear lacquer is employed.

8. A process according to any one of the preceding claims, characterised in that a radiation-hardenable coating medium is used as the lacquer.

9. A process according to any one of claims 1 to 7, characterised in that a lacquer which is hardenable by stoving is employed.

10. A process according to claim 9, characterised in that a hot-sprayable lacquer is employed, particularly a clear lacquer, based on
I. 45 to 85 % by weight of one or more polyesters which are of branched structure and are substantially free from aromatic structural units, with a number average molecular weight of 350 to 3000 and a polydispersity less than 3.5, and
II. 10 to 40 % by weight of one or more aminoplast resins and/or blocked di- and/or polyisocyanates as crosslinking agents, and
0 to 20 % by weight of one or more reactive thinners, and
0 to 10 % by weight of one or more organic solvents,
wherein the lacquer has a viscosity of 100 to 1000 mPa.s, measured in a rotating viscometer at 70°C and at a shear rate of 235 sec⁻¹.

11. An apparatus suitable for carrying out the process according to any one of claims 1 to 10, having a supply vessel (1) for lacquer, a spraying element (7) connected thereto which is situated in a spray booth (10) equipped with devices for feeding (12) and with a line (14) for the discharge of an air current, a deposition zone (11) for overspray having deposition surfaces, having liquid/gas separation devices (15,16) situated outside the spray booth (10) and connected to the line (14), wherein the deposition surfaces of the deposition zone (11) are free from devices for wet scrubbing, the spray booth (10) and the liquid/gas separation devices (15,16) are provided with collection points (13,13a,13b) for overspray, from which lines (22,24,29) lead to the supply vessel (1), and that a line (19) is provided for feeding back to the spray booth (10), in circulation, the air discharged from the spray booth (10) through the line (14), characterised in that the deposition surfaces of the deposition zone (11) can be heated.

12. An apparatus according to claim 11, characterised in that the line (22) which discharges from the collection points (13,13a,13b) leads to a collecting tank (23) which is connected to the supply tank (1).

13. An apparatus according to either one of claims 11 and 12, characterised in that one or more heat exchangers (26,28) and/or lacquer filters (27) are included in the line (22,24,29) connecting the collection points (13,13a,13b) and the supply tank (1).

## Revendications

1. Procédé de pulvérisation de laques monocomposants liquides dans une cabine de peinture à travers laquelle on souffle un flux d'air, avec collecte et évacuation de la surdispersion qui se produit, une partie de la surdispersion étant collectée à l'intérieur de la cabine de peinture sur une ou plusieurs surfaces réceptrices sans lavage humide et évacuée de cette cabine de peinture ; on sépare la quantité restante de la surdispersion du flux d'air sortant de la cabine de peinture au moyen d'un ou de plusieurs groupes de séparation liquide/gazeux et qu'on l'évacue de ces groupes, on réunit à de la laque neuve la surdispersion évacuée de la cabine de peinture et celle séparée du flux d'air, sans autre préparation, pour la réutilisation directe, et on renvoie dans la cabine de peinture, en circuit fermé, le flux d'air sortant de la cabine de peinture, caractérisé en ce qu'on pulvérise une laque monocomposant pulvérisable à chaud à des températures allant jusqu'à 90°C, ayant une teneur en solides de plus de 90 % en poids, on évacue plus de 80 % en poids de la surdispersion et on travaille dans une cabine de peinture possédant des surfaces réceptrices chauffées.

2. Procédé selon la revendication 1, caractérisé en ce qu'on filtre la surdispersion évacuée de la cabine de peinture avant de la mélanger à de la laque neuve.

3. Procédé selon une des revendications 1 et 2, caractérisé en ce qu'on pulvérise la laque à l'aide d'un solvant super-critique, notamment à l'aide de dioxyde de carbone.

4. Procédé selon une des revendications précédentes, caractérisé en ce qu'on travaille avec une laque comportant une proportion d'un ou de plusieurs solvants organiques liquides de moins de 5 % en poids.

5. Procédé selon une des revendications précédentes, caractérisé en ce qu'on travaille avec une laque ayant une teneur en un ou plusieurs solvants organiques liquides ayant un point d'ébullition ou un intervalle de point d'ébullition de 170°C ou plus.

6. Procédé selon une des revendications 1 à 3, caractérisé en ce qu'on travaille avec une laque qui est exempte de solvants organiques liquides.

7. Procédé selon une des revendications précédentes, caractérisé en ce qu'on travaille avec un vernis incolore monocomposant.

8. Procédé selon une des revendications précédentes, caractérisé en qu'on utilise comme laque un milieu de revêtement durcissable par rayonnement.

9. Procédé selon une des revendications 1 à 7, caractérisé en ce qu'on travaille avec une laque durcissable par cuisson.

10. Procédé selon la revendication 9, caractérisé en ce qu'on travaille avec une laque pouvant être pulvérisée à chaud, notamment un vernis incolore, à base de
I. 45 à 85 % en poids d'un ou plusieurs polyesters qui sont de construction ramifiée et sont sensiblement exempts d'unités structurelles aromatiques, ayant un poids moléculaire moyen en nombre de 350 à 3000 et une polydispersité inférieure à 3,5, et
II. 10 à 40 % en poids d'une ou plusieurs résines aminoplastes et/ou un ou plusieurs di- et/ou polyisocyanates bloqués en tant que réticulant, et
0 à 20 % en poids d'un ou plusieurs diluants réactifs, et
0 à 10 % en poids d'un ou plusieurs solvants organiques,
où la laque présente une viscosité de 100 à 1000 mPa.s, mesurée au viscosimètre à rotation à 70°C et un gradient de cisaillement de 235s⁻¹.

11. Installation, appropriée pour la mise en oeuvre du procédé selon une des revendications 1 à 10, comprenant un réservoir (1) pour la laque, un organe de pulvérisation (7) qui y est raccordé, et qui se trouve dans une cabine de peinture (10) qui est équipée de dispositifs pour l'introduction (12) d'un flux d'air et une conduite (14) pour l'évacuation du flux d'air, d'une zone de réception (11) pour la surdispersion, équipée de surfaces de réception, des dispositifs de séparation liquide/gazeux (15, 16) placés à l'extérieur de la cabine de peinture (10) et reliés à la conduite (14), les surfaces réceptrices de la zone de réception (11) étant exemptes de dispositifs de lavage humide, la cabine de peinture (10) et les dispositifs de séparation liquide/gazeux (15, 16) étant équipés de points collecteurs (13, 13a, 13b) pour la surdispersion, d'où partent des conduites (22, 24, 29) menant au réservoir (1) et en ce qu'une conduite (19) servant à renvoyer en circuit fermé dans la cabine de peinture l'air qui a été évacué de la cabine de peinture (10) par la conduite (16), caractérisée en ce que les surfaces réceptrices de la zone de réception (11) peuvent être chauffées.

12. Installation selon la revendication 11, caractérisée en ce que la conduite (22) qui part des points collecteurs (13, 13a, 13b) conduit à une cuve collectrice (23) qui est reliée au réservoir (1).

13. Installation selon une des revendications 11 et 12, caractérisée en ce qu'un ou plusieurs échangeurs de chaleur (27, 28) et/ou filtres à laque (27) sont intercalés dans la conduite (22, 24, 29) qui relie les points collecteurs (13, 13a, 13b) au réservoir (1).
